(19) Europäisches Patentamt / European Patent Office / Office européen des brevets

(11) EP 2 069 357 B1

(12) EUROPEAN PATENT SPECIFICATION

(45) Date of publication and mention of the grant of the patent: **26.08.2015 Bulletin 2015/35**

(21) Application number: **06843903.3**

(22) Date of filing: **21.12.2006**

(51) Int Cl.: ***C07D 493/04*** *(2006.01)*

(86) International application number: **PCT/KZ2006/000016**

(87) International publication number: **WO 2008/035958 (27.03.2008 Gazette 2008/13)**

(54) **METHOD FOR PRODUCTION OF HYDROCHLORIDE 1(10) BETA-EPOXY-13-DIMETHYLAMINO-5,7ALPHA,6,11BETA (H)-GUAIA-3(4)-EN-6,12-OLIDE, THE LYOPHILIZED ANTITUMOR PREPARATION "ARGLABIN"**

VERFAHREN ZUR HERSTELLUNG VON 1(10)-BETA-EPOXY-13-DIMETHYLAMINO-5,7-ALPHA-6,11-BETA-(H-GUAIA-3(4)-EN-6,12-OLIDHYDROCHLORID, DER LYOPHILISIERTEN ANTITUMORZUBEREITUNG ARGLABIN

PROCEDE DE PRODUCTION DE 1(10) BETA-EPOXY-13-DIMETHYLAMINO-5,7ALPHA,6,11BETA (H)-GUAIA-3(4)-EN-6,12-OLIDE HYDROCHLORURE, PREPARATION ANTITUMORALE LYOPHILISEE 'ARGLABINE'

(84) Designated Contracting States: **DE**

(30) Priority: **19.09.2006 KZ 061041**

(43) Date of publication of application: **17.06.2009 Bulletin 2009/25**

(73) Proprietor: **Adekenov, Sergazy Mynzhasarovich Karaganda, 100019 (KZ)**

(72) Inventor: **Adekenov, Sergazy Mynzhasarovich Karaganda, 100019 (KZ)**

(74) Representative: **von Füner, Nicolai et al**
**v. Füner Ebbinghaus Finck Hano**
**Patentanwälte**
**Mariahilfplatz 2&3**
**81541 München (DE)**

(56) References cited:
**WO-A-98/28303 WO-A-2006/012824**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 069 357 B1

## Description

The invention relates to technology for production of new medicinal preparations from the natural raw material, namely antitumor preparation "Arglabin" and can be used for chemotherapy of malignant tumors and dilating of range of immunomodulating preparations.

The sesquiterpene lactone arglabin derivative, 1(10)β-epoxy-13-dimethylamino-5,7α,6,11β(H)-guaia-3(4)-en-6,12-olide hydrochloride (**1**) synthesized from 1(10)β-epoxy-5,7α,6β(H)-guaia-3(4),11(13)-dien-6,12-olide (**2**), last discharged from endemic plant *Artemisia glabella Kar. et Kir* is used as an antitumor preparation.

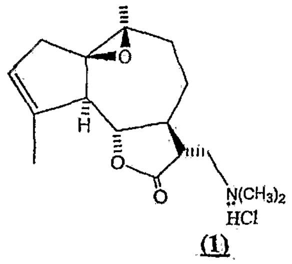
(1)

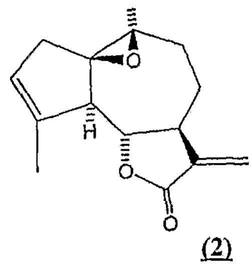
(2)

The method for production of 1(10)β-epoxy-13-dimethylamino-guaia-3(4)-en-6,12-olide hydrochloride lyophilized antitumor preparation "Arglabin" from the natural raw material (Patent of the Republic of Kazakhstan No 10913, cl. A61K 31/343, 31/336, A61P 35/00, 15.03.2004) is known. The method includes extraction of the natural raw material (an above-ground part of endemic plant *Artemisia glabella Kar. et Kir.*), purification of the sum of extracted substances (resin) from worthless products, separation resin into individual components by the method of column chromatography with obtaining technical arglabin, witch further recrystallization, amination and hydrochlorination with production dimethylamino derivative.

The basic disadvantage of the method is significant duration of process.

Closest to the method according to the present invention is the method for production lyophilized arglabin, having antitumor and immunomodulating activities, from the natural raw material as which use flower baskets and leaves of endemic plant - *Artemisia glabella Kar. et Kir.* (Preliminary patent of the Republic of Kazakhstan No 5277, cl. C07D 307/93, A61K 31/365, 15.10.1997). Initially dry grinded natural raw material is treated in an extractor by solvent as chloroform, and then extracted substances (resin) are purified from worthless products by treatment with water-alcohol mixture. The resin obtained at the stage of purification is separated into individual components by the method of column chromatography on silica gel for 72 hours. The fractions containing arglabin are separated at elution with benzene. Benzene is evaluated and technical arglabin is received and further subjected to recrystallization, amination and hydrochlorination with production dimethylamino derivative. Duration of a synthesis stage is 48 hours.

Amination is carried out by dimethylamine with receiving of a solution containing aminoarglabin. Then the alcohol solution of aminoarglabin is barbotaged by hydrogen chloride and technical dimethylaminoarglabin hydrochloride is produced. The received product is purified by recrystallization with subsequent drying and lyophilization.

The method with using of chloroformic extraction of the natural raw material has the following disadvantages:

- chloroform is an expensive and toxic solvent;
- duration of extraction is 6 - 8 hours;
- using of the method of column chromatography at separation resin is long on time (72 hours), and used eluent benzene is also a toxic solvent;
- duration of the stage of synthesis dimethylamino derivative is about 48 hours.

WO 2006/012824 A discloses a method for producing dimethylaminoarglabin hydrochloride. After air drying, useless substances are eliminated, then extraction and chromatography are carried out, subsequently amination is carried out and then the hydrochloride is formed in a reactor without repeated intermediate isolations, such as evaporation, filtration or dehydration, and with only one change of organic polar solvents.

The purpose of the present invention is development of a method for production of an antitumor preparation on the basis of the natural sesquiterpene lactone **(1)** which excludes using of toxic reagents and provides significant reduction of process duration.

The specified technical result is provided in a method for production of 1(10)ß-epoxy-13-dimethylamino-5,7α,6,11ß(H)-guaia-3(4)-en-6,12-olide hydrochloride, the lyophilized antitumor preparation having radio-sensitizing and immunomodulating activities, including extraction of the natural raw material, endemic plant *Artemisia glabella Kar. et Kir.*, separation received resin on individual components with receiving technical arglabin from which dimethylaminoarglabin hydrochloride is synthesized, with its subsequent purification, drying and lyophilization; and extraction is carried out with using as extracting agent of fluid carbon dioxide at pressure 150 ± 2 · 105 Pa and temperature 60°C ± 0,5, purification of the resin is carried out by treating it with a water-alcohol mixture in the ratio 1 : 2 at a temperature of 70°C, settling the filtrate in a cooler for 24 hours at temperature of 10 - 15°C with a separation of the filtrate from the residual, subjecting the residual to water-alcohol processing for three times at a ratio of 1 : 2, combining and evaporating the filtrate up to 1/5 starting volume, to the obtained evaporated filtrate ethyl acetate is added in the ratio 1 : 1, the received solution is transferred into a separator and stirred for 5 minutes before complete separation of layers, the resin separation into individual components is carried out by the method of preparative fluid chromatography with application of reversed phase $C_{18}$, for the synthesis of dimethylaminoarglabin hydrochloride the technical arglabin is solved in acetonitrile at weight ratio 1 : 10 with the addition dimethylammonium dimethylcarbamat in the molar ratio 1 : 1,62.

For approbation of the production technology of isolation arglabin with use of equipment firms "Flavex" (Germany) extraction 600 kg of the natural raw material was carried out under the conditions of extraction set on the method according to the present invention. Results had shown that the arglabin content in the $CO_2$-extrac in 2 times was higher, than in the chloroform extract received on the known method. The cost price of arglabin isolation from the $CO_2$-extract is lower due to economy of the solvents. The method of $CO_2$-extraction promotes selective extraction arglabin from the natural raw material and is accompanied by natural decrease of the residual content of arglabin in waste material.

Experiment results on selection of arglabin isolation optimal conditions are given in Table 1. It shows, that the greatest content of arglabin in the $CO_2$-extract of 28,5 % is reached at pressure $150 \cdot 10^5$ Pa, a temperature 60°C and time of extraction of 75 minutes. Charge $CO_2$ is 100 kg/h.

The arglabin content in the extract after C02-extraction was determined by the method of high performance liquid chromatography (HPLC). Analysis was carried out on the liquid chromatograph of firm HEWLETT PACKARD® Agilent 1100 Series with the UV-detector under following conditions:

- analytical column filled with sorbent Zorbax CB-$C_{18}$ 4,6 x 150 mm, with particle size 5 µm;
- composition of mobile phase of acetonitrile : water in the ratio 50:50;
- detecting at wave length of 204 nm;
- room temperature of the column;
- speed of the mobile phase of 0,75 ml/min;
- volume of introduced assay 20 µl.

Table 1. The arglabin content in the $CO_2$-extract in dependence on conditions of extraction

| No experi ment | Extraction pressure, $10^5$ Pa | Extraction temperature, °C | Speed of stream $CO_2$, kg/h | Charge $CO_2$, kg/h | Extraction time, min | Yield of extract | | Arglabin content in $CO_2$- extract, % |
|---|---|---|---|---|---|---|---|---|
| | | | | | | g | % | |
| 1 | 100 | 50 | 80 | 45 | 25 | 31,3 | | 24,0 |
| 2 | 150 | 50 | 80 | 102 | 64 | 126 | 3,15 | 26,3 |
| 3 | 200 | 50 | 80 | 100 | 70 | 233 | 4,66 | 20,0 |
| 4 | 150 | 60 | 80 | 100 | 75 | 157 | 3,14 | 28,5 |

Exact shot of a researched sample of the $CO_2$-extract was solved in 10 ml of the mobile phase at careful stirring. The received solution was quantitatively transferred to a measured flask capacity of 25 ml, the volume of the solution was lead up to a label and stirred.

The received solution was filtrated through a membrane filter with pore size of 0,45 µm. On 20 µl of the filtrate and a solution of reliable arglabin sample were chromatographed in the conditions described above.

The arglabin quantitative content (X) in percentage was determined by the comparison method with the external standard by the formula:

$$X = \frac{S_1 \cdot m_o \cdot 25 \cdot 100}{S_o \cdot m_1 \cdot 25},$$

where

$S_1$ - an average value of arglabin peak areas on chromatograms of tested solution;
$S_0$ - an average value of arglabin peak areas on a chromatogram of the solution of the reliable sample;
$m_0$ - arglabin shot of the reliable sample, g;
$m_1$ - a shot of the $CO_2$-extract, g;
25 - dilution.

The data under the arglabin content in assay of the $CO_2$-extract of endemic plant and in the waste material are shown in Table 2.

Results of the carried out experiments has shown, that the maximal yield among the first separators is registered at a regimen: pressure of $150 \cdot 10^5$ Pa and temperature of 60°C. Thus the yield of target substance is increased and the extraction time is considerably decreased, than at the known chloroform extraction.

Received $CO_2$-extract was purified from a lipide fraction, protein substances and chlorophyll by treating with a water-alcohol mixture in the ratio 1 : 2 at temperature of 70°C, with settling a filtrate in a cooler for 24 hours at temperature of 10 - 15°C. The treated mixture was filtrated, then collected in a separate container, the residual on the filter was subjected to water-alcohol processing three times at the ratio 1 : 2, filtrates were combined and evaporated up to 1/5 starting volumes. To the evaporated filtrate ethyl acetate was added in the ratio 1 : 1, received solution was transferred into a separator and stirred for 5 minutes before complete separation of layers. Layers were separated; the inferior aqueous layer was extracted with ethyl acetate three times. Ethyl acetate extractions were combined and evaporated to dryness. The yield of the purified extract is 73,3 % in relation to the concentrated extract.

Table 2. Definition of arglabin content in samples after $CO_2$-extraction by method HPLC.

| No | Name of sample | Parameters of extraction | Description | Arglabin content, % from total extract | Quantity of sample, g | Yield (of all) | | Arglabin content | | Arglabin content (in relation to absolutely dry raw material) |
|---|---|---|---|---|---|---|---|---|---|---|
| | | | | | | % | g | % | g | |
| 1 | Endemic plant extract Separator 1 | $150 \cdot 10^5$ Pa / 70°C 3 hours | Pasty yellow substance | 67,26 | 1,71 | 5,12 | 76,79 | 15,58 | 11,96 | |
| 2 | Endemic plant waste material | $150 \cdot 10^5$ Pa / 70°C 3 hours | Fine green - grey powder | 4,49 | 20,16 | 89,10 | 1336,53 | 0,06 | 0,8 | 0,06 |
| 3 | Endemic plant extract Separator 1 | $150 \cdot 10^5$ Pa/60°C 3 hours | Pasty yellow substance | 73,62 | 2,82 | 6,85 | 102,75 | 12,74 | 13,09 | |
| 4 | Endemic plant waste material | $150 \cdot 10^5$ Pa / 60°C 3 hours | Fine green - grey powder | 4,55 | 15,78 | 90,40 | 1355,94 | 0,06 | 0,81 | 0,06 |

Purified extract was transferred to the preparative chromatography stage for selection of individual substance on the preparative fluid chromatography apparatus "Super Pure - 350".

Chromatographic fractionation was carried out by means of isocritical system of solvents (acetonitrile-water at the ratio 1 : 1) on reversed phase $C_{18}$ with discrete device. The range of working pressure is from $50 \cdot 10^5$ up to $320 \cdot 10^5$ Pa, the temperature from 5 up to 80°C, speed of co-solvent flow from 20 up to 350 ml/min in system, the column internal diameter of 10 sm. Purity technical arglabin received by means of preparative fluid chromatography apparatus "Super Pure-350" is 99 %.

Use of the preparative fluid chromatography method with application of reversed phase $C_{18}$ allows carrying out separation into individual components for 3 hours while in the known method on separation by the column chromatography method 72 hours are required. Besides at the column chromatography the toxic solvent benzene, excluded in the method according to the present invention, is used.

1(10)β-Epoxy-13-dimethylamino-5,7α,6,11β(H)-guaia-3(4)-en-6,12-olide hydrochloride **(<u>1</u>)** was synthesized for the further reception of the water-soluble form of lactone dimethylamino derivative hydrochloride. Initial substance, sesquiterpene lactone arglabin **(<u>2</u>),** was solved in acetonitrile at the weight ratio 1 : 10. Then in acetonitril solution dropwise at stirring for 10 minutes dimethylammonium dimethylcarbamat was added in the molar ratio of initial substance to dimethylammonium dimethylcarbamat, equal to 1 : 1,62. After complete conversion of initial substance (during 25 minutes) acetonitrile and dimethylammonium dimethylcarbamat residues are evaporated on the rotary evaporator.

After end of the process in the reaction mixture the ethyl acetate was added cooled up to temperature 5 - 15°C in the ratio 1 : 2. Barbotage process by gaseous hydrogen chloride (HCl) was carried out at slow stirring at temperature of 12 - 15°C ± 0,3 (the beginning of reaction), the reaction was completed at temperature of 16 - 18°C ± 0,5. Optimal duration of the reaction was 15 minutes. Feeding of gas was intercepted at achievement of pH range 5,2-5,5 ± 0,05. After end of the process the reaction mixture was transferred to a flask of the rotary evaporator capacity 21, excess of ethyl acetate and hydrogen chloride were evaporated to syrup state. To the obtained solution absolute ethyl acetate (99,9 %) was flowed preliminarily cooled up to temperature 10°C and aggressively shook up before precipitation of white crystals. The flask with crystalline mass was kept in a cooler at temperature 12 - 15°C for 1 hour. The precipitated crystals were filtered off through filter Shotta and flushed on the same filter by cooled ethyl acetate. The filtered crystals after recrystallization from ethyl acetate were dried up on the rotary evaporator at temperature of 40 - 70°C for 1 hour. Final drying of crystals were carried out in the vacuum drying at temperature of 40 - 50°C, pressure of 5 - 10 mm Hg column up to a weight loss at drying not higher 0,5 %. The obtained product represents a white crystalline powder of dimethylaminoarglabin hydrochloride **(<u>1</u>)** with yield of 90 %.

In the method according to the present invention at the stage of dimethylaminoarglabin hydrochloride synthesis using dimethylammonium dimethylcarbamat instead of dimethylamine in the known method has allowed to reduce duration of synthesis stage from 48 to 3 hours.

Substance of dimethylaminoarglabin hydrochloride **(<u>1</u>)** is used for production of the lyophilized medicinal form.

Final product (substance) was solved in water for injections at the rate of: 2 g of dry substance per 100 ml of water. Obtained 2 % solution was lyophilized by the following method. Solution was filtrated by using filtration device "Sartorius" through sterile capsule "Sartobran" with pore size 0,45 μm, 0,2 μm accordingly in sterile glass container. Obtained filtrate by means of automatic device "Rose - 2" was poured in ampulas on 2 ml and dried in liophilizator LZ-45. Dosed in ampulas solution was seated in cartridges. Cartridges were covered with sheets of sterile parchment and seated into low-temperature tables "Debri" (Holland) for freezing at -40°C, not less than for 12 hours. After end of freezing process the cartridges with ampulas were placed on sublimator shelves and seated in a vacuum boiler of sublimator LZ-45 for carrying out of sublimation drying process. In 1 hour after dipping shelves in the boiler of the sublimator a vacuum pump was connected, further on achievement of necessary vacuum value (not less than 5 - 6 MPa), approximately in 2 hours after the beginning of drying process, preheating sublimator shelves was begun. Shelves were heated up gradually up to +55 (+5°C) by adding from 0°C up to +55°C on 5°C per each hour of sublimation drying. On subzero temperature from 0°C up to -30°C was added on 10°C. Transferring of a product from subzero temperature to positive temperature was carried out on the 12th - 13th hour of drying. Final temperature of the product should not exceed +50, +52°C. Duration of drying was 24 hours.

Authenticity of the preparation is determined on melting point, the IR-spectrum, HPLC-analysis.

Apparently from the above-stated data, the method according to the present invention is carried out in the conditions corresponding to norms GMP, namely:

- fluid carbon dioxide is nontoxic extragent;
- isolation of individual substance is carried out on preparative fluid chromatography apparatus "Super Pure-350" with application of reversed phase $C_{18}$;
- at synthesis stage modifying with use dimethylammonium dimethylcarbamat is carried out.

Substance of the preparation, 1(10)β-epoxy-13-dimethylamino-5,7α,6,11β(H)-guaia-3(4)-en-6,12-olide hydrochloride **(1)**, was studied on antitumor activity on 11 strains of experimental tumors in experiment.

To arglabin the following strains of interweave tumors are sensitive: lung carcinoma, Ehrlich's solid tumor, mamma adenocarcinoma Ca - 755, sarcoma 37, lymphocytic leukemia P - 388, lymphoid leukemia L - 1210, sarcoma M - I, Walker carcinosarcoma, alveolar mucous cancer of liver PC - 1 of rats at intraperitoneal and intratumoral introduction of the preparation in maximal tolerance doses (MTD).

Arglabin also shows efficiency in the relation to interweave tumors, resistant to prospidine, rubomicine and sarcolyzine.

It is fixed, that the preparation "Arglabin lyophilized" efficiently influences in the initial stages of metastasis formations, i.e. reduces an opportunity of their occurrence.

Arglabin has ability to optimize oxidation-reduction processes and a metabolism in a connective tissue.

Arglabin immunomodulating activity is determined which depends on a dose of the preparation and shows primary influence on the T-cellular part of immunity.

Arglabin has a property to correct immunodepressive effect of cytostatic cells.

It is revealed that arglabin is a competitive inhibitor of RAS-oncoproteins pharnesilis, reduces an expression of RAS-genes and ATP content, invokes tumor cells apoptosis.

The preparation has a sufficiently high parameter of time-retention in an organism (almost 22 hours). Arglabin quickly penetrates from central camera (blood) into peripheral tissues. During the first hour the greatest concentrations of the preparation are framed in lien and lung, in 3 hours it is collected also in liver and skeletal muscles.

The preparation in as much as possible tolerable doses does not influence morphological structure of peripheral blood and an osseous tissue, does not break a function state of liver, kidneys, cardiovascular system, breathing, peripheral nervous system.

At intravenous, intraperitoneal, intramuscular, hypodermic introduction the preparation does not render topical-irritating action. Allergenic and pyrogenic properties are not revealed. Arglabin also has no embryotoxic, teratogenic and mutafacient properties.

Clinical research «Arglabin lyophilized» on the first phase was carried out on 53 patients with III-IV stages of malignant process of 11 localizations. The unitary dose was introduced from 240 up to 480 mg per day. The preparation was introduced intravenously stream of 2 % aqueous solution. At presence at patients of an ascites and a pleuritis 2 % solution was introduced into a cavity in the dose of 480 mg (in physiological salt solution).

It is revealed that the preparation in advisable doses has no toxicity, does not render oppressing action on hemopoiesis, does not break function of internals, peripheral nervous system.

The second phase of clinical tests of the preparation was carried out on 72 patients with IV stage of tumor process of 11 localizations (a cancer of liver, lung, stomach, mamma, rectum, esophagus, ovary, pancreas or submandibular sialaden, a lymphosarcoma, etc.).

As a result of the second phase of clinical tests the following results were received:

- partial regress of a tumor 32 (44,4 %) patients;
- stabilization of process 12 (16,7 %) patients;
- progressing of process 28 (38,9 %) patients.

The estimation of objective effect of the preparation monochemotherapy was carried out by the criterion recommended by World Health Organization (WHO) and International Anticancerogenic Union. The best results of arglabin chemotherapy were received at an initial cancer of liver, mamma, lung and ovary.

Treatment of patients by a disseminated mamma cancer was carried out in three groups generated by the randomization method:

I group - arglabin monochemotherapy (15 patients);
II group - arglabin in combination with CMF schema (15 patients)
III group - chemotherapy under CMF schema (15 patients) - control.
The single dose arglabin on the average was 240 mg, total dose was 4,8 g.

The high parameter of particulate regression and stabilization of the process was achieved in II group (arglabin + CMF).

The survival rate of patients by a disseminated cancer of mamma (calculations on E. Kaplan - P. Meier) was investigated.

In group of the patients receiving only «Arglabin lyophilized» the six-monthly survival rate were equal 57,8 %, year survival rate was 3,9 %. The median of survival rate was 6,3 months. In group of the patients receiving only polychemotherapy under CMF schema the said parameters was accordingly 72,0 % and 17,3 %, and the median of survival rate was 7,6 months. Higher parameters were observed in the group receiving arglabin in combination with

polychemotherapy under CMF schema, where six-monthly survival rate was 100 %, year survival rate was 39,1 %. The median of survival rate was 10,9 months (P < 0,05).

After treatment, at the end of radial therapy and before operation, authentically greater decrease of a tumor volume was observed in the first and second groups (P < 0,001), than at patients of the third group. The pathomorphism of III-IV degree tumor in the first and second groups was accordingly 64 ± 9,8 % and 50 ± 0,2 %, whereas in the third group was 37 ± 9,5 %.

Cumulative parameters of survival rate (calculations on E. Kaplan - P.Meier) were determined. The three-year survival rate among patients of the first and third groups appeared identical and was accordingly 23,9 ± 7,8 % and 24,4 ± 7,8 %, whereas in the second group was 45,2 ± 9,1 %.

The same was observed at studying non-recurrent survival rate. Thus in the first and third groups 3 year survival rate was accordingly 5,2 ± 4,1 % and 5,2 ± 4,1 %, in the second group was 32,5 ± 8,6 %.

The median of the survival rate in the first and third groups was identical (3 ± 0,03 year), in the second group said parameter was authentic above (7,4 ± 0,2 year), than in the previous two groups.

At use of "Arglabin lyophilized" on 34 patients with disseminated forms of tumors and developed fastness to chemicals in combination with chemotherapy at the incurable and weakened patients (Karnovskiy index of 60-70 %) the positive effect in 44 % was received. These results testify about chemo-sensitizing activities of "Arglabin lyophilized". "Arglabin lyophilized" is a preparation of choice for the weakened and incurable patients when other kinds of standard antitumor therapy are not acceptable.

Researches on 55 patients with the initial liver cancer distributed on 3 bunches were carried out:

1 group (control) - receiving polychemotherapy (26 patients).
2 group - receiving intravenous «Arglabin lyophilized» in a single dose at the rate of 5 mg/kg of weight (185 $mg/m^2$) for 20 - 21 days (17 patients).
3 group - receiving intravenous «Arglabin lyophilized» at the rate of 5 mg/kg of weight (185 $mg/m^2$) in combination with fluorouracil of 600 $mg/m^2$ for 1,8 day (12 patients).

Efficiency of chemotherapy was determined by the WHO criterion. The survival rate was calculated by acturial method.

The year survival rate in the first and second groups of the patients receiving polychemotherapy by standard drugs and only «Arglabin lyophilized» was accordingly 32,2 % and 32,3 %. In the third group the survival rate authentically exceeded parameters of first two groups and was 55,1 % (P <0,05).

Definition of a median of survival rate also had shown that it was higher in the third group (15,3 months), while in the first and second ones were accordingly 7,85 and 8,45 months.

45 Patients with lung cancer basically with T3N1MO stage of disease were introduced «Arglabin lyophilized», from them 6 patients after a trial thoracotomy, 21 initial patients, 24 repeated patients after applied before courses of radial and chemotherapies. At all patients histological acknowledgement of the diagnosis took place: epidermoid carcinoma at 16, small-cell carcinoma at 12, glandular cancer at 10 and carcinoma simplex at 7 patients.

I. The basic group: «Arglabin lyophilized» on 240 mg from 1st to 20th days, methotrexat on 25 mg in 1st, 4th, 8th, 11th days, endoxan on 1,0 g in 1st, 5th, 9th days (15 patients).
II. Control group 1: «Arglabin lyophilized» on 240 mg from 1st to 20th days (15 patients).
III. Control group 2: methotrexat on 25 mg in 1st, 4th, 8th, 11th days, endoxan on 1,0 g in 1st, 5th, 9th days (15 patients).

At carrying out of "Arglabin lyophilized" chemotherapy at a part of patients improvement of the general state, decrease of tussis and breast pains estimated basically in 1 point were marked. By the end of treatment at overwhelming number of patients stabilization of process was achieved. At two patients of first control group progressing disease was marked.

The part of patients had arrived in a hospital repeatedly a month later after the termination of the first course of treatment. At control inspection practically at all patients stabilization of process continued to be determined.

Efficiency of "Arglabin-lyophilized" preparation as radio-sensitizing agent for radiation and chemo-radiation therapies at 20 patients with topical mamma cancer was determined. Complete and particulate regression of a tumor were achieved at 19 of 20 patients in the researched group (95,5 %), and at 4 (20,0 %) from them complete tumors resorption was marked. In the control group the objective antitumor effect was 70 %. For more objective assessment of "Arglabin lyophilized" treatment at histological research of an operative material the degree of a medical pathomorphism in a tumor and metastatic modified lymphonoduses were determined. The third degree of a medical pathomorphism (a necrosis, a fibrosis, individual degenerately modified cells of a cancer) was fixed at 11 (55 %) patients of the researched group and at 4 (20 %) patients of the control group. The complete and expressed antitumor effect by morphological criterion were marked at 14 (70 %) patients receiving "Arglabin-lyophilized", that in 3,5 times higher in comparison with

the control group (20 %). "Arglabin-lyophilized" in a single dose of 5 mg/kg introduced intravenously stream 15 minutes prior to radial therapy in a day had no side effects and was well tolerated by patients.

Thus, «Arglabin lyophilized» has the expressed sensitizing effect, authentically enlarging frequency of objective regressions and frequency of the expressed medical pathomorphism (III-IV degrees) both in an initial tumor, and in metastatic modified lymphonoduses.

Research of the interferon (IFN) status and cytokine profile at 18 women with mamma cancer in dynamics before and after radiation therapy with application of "Arglabin-lyophilized" preparation testifies that it recovers the synthesis mechanism of the following cytokines: IFN-$\gamma$, Il (interleukin) -2, anti-inflammatory cytokine Il-4, TNF (tumor necrosis factor)-$\alpha$, Il-1$\beta$.

At the surveyed patients after treatment by "Arglabin-lyophilized" preparation it was marked:

1. Drop spontaneously developed IFN at all patients.
2. Rising up to norm of ability to production IFN-$\alpha$ and IFN-$\gamma$ on the average at 40 % of patients.
3. Sensitization to preparations IFN-$\alpha$ and IFN-$\gamma$ at 44 % and 11 % of patients, accordingly.
4. Sensitivity to immunomodulating preparations (ridostine, cycloferon and amicsine) was improved in 30 - 40 % of cases, and to the researched preparation "Arglabin lyophilized" in 40 - 50 % of cases.

Also it was shown that at all patients with <u>positive clinical effect</u> at the therapy including "Arglabin lyophilized" preparation the expression of genes IFN-$\gamma$ and 11-2 (50 %) or only 11-2 (50 %) was marked. Thus genes activity of specified cytokines at other patients was marked only in 27 % and 73 % of cases, accordingly. It is known that these cytokines are developed by Th1-lymphocytes, and depressing of Th1 activity with drop of development IFN-$\gamma$ or I1-2 results in more serious course of disease. Therapy with application "Arglabin-lyophilized" by these parameters can be effective.

It was marked that at <u>positive clinical effect</u> of therapy with application of "Arglabin-lyophilized" preparation mRNA Il-12-cytokine, participating in development Th1-type immune answer, was defined at 60 % of patients, that in 2 times is more often, than at other patients.

The carried out researches also had shown that mRNA I1-1$\beta$ was defined practically at all surveyed patients after treatment. Thus activity mRNA Il-6 and I1-8 was marked at <u>positive clinical effect</u> at 70 % and 60 % of patients, accordingly, and at stabilization of a state at 80 % and 70 % of patients, accordingly.

Simultaneously with research of IFN status and an assessment of an expression of genes cytokines on a level of synthesis their mRNA at the same patients with mamma cancer were determined the cytokine contents (method of enzyme multiplied immunoassay) in serum of blood and at cultivation of blood cells.

<u>After a course of the carried out treatment with «Arglabin lyophilized»</u> normalization or the tendency to normalization of quantity investigated cytokines, determined in serum of blood, at 20 - 40 % of the surveyed women was marked. Thus also it was shown that at 10 - 30 % of patients the cytokine contents increased in serum of blood that could testify to an activation of immune system cells.

The data obtained during research of a state of interferon system and cytokines at patients with mamma cancer during clinical test of "Arglabin lyophilized" preparation, are testified about high immunomodulating activities of the investigated preparation at the given group of patients.

Advantage of the method according to the present invention for production of antitumor preparation "Arglabin":

- presence of the industrial raw-material base; the endemic plant (*Artemisia glabella Kar. et Kir.*) is cultivated in a pilot farm of the Karaganda pharmaceutical factory (Kazakhstan, Karaganda) on the area 5,6 ha with annual effecting 6,0 t of dry mass for the subsequent processing. In the long term till 2010 annual production raw material is planned to finish up to 40 t per one year,
- target substance - sesquiterpene lactone 1(10)$\beta$-epoxy-5,7$\alpha$,6$\beta$(H)-guaia-3(4),11(13)-dien-6,12-olide **(<u>2</u>)** is extracted from the natural raw material with application of nonpolluting technology with use of nontoxic extragent fluid carbon dioxide that meets to norms GMP as excludes extraction by toxic solvent chloroform;
- the yield of target substance at extraction by fluid carbon dioxide in 2 times is higher, than at chloroformic extraction in relation to air-dry raw material;
- the method according to the present invention provides significant decrease of process duration: time of extraction reduced in 2 times (from 6 hours to 3 hours), chromatographic fractionation in 36 times (from 72 hours to 3 hours); stages of synthesis in 16 times (from 48 hours to 3 hours);
- isolation and purification of individual substance is carried out on apparatus preparative fluid chromatography "Super Pure - 350" with application of reversed phase $C_{18}$, thus use of toxic solvent benzene is excluded;
- the synthesis stage of substance 1(10)$\beta$-epoxy-13-dimethylamino-5,7$\alpha$,6,11$\beta$(H)-guaia-3(4)-en-6,12-olide hydrochloride **(<u>1</u>)** is carried out with use dimethylammonium dimethylcarbamat instead of dimethylamine;
- results of clinical tests have shown high radio-sensitizing, immunomodulating activity of the antitumor preparation

"Arglabin" received by the method according to the present invention.

## Claims

**1.** method for the production of 1(10)ß-epoxy-13-dimethylamino-5,7$\alpha$,6,11ß(H)-guaia-3(4)-en-6,12-olide hydrochloride, comprising the extraction of the natural raw material endemic plant *Artemisia glabella Kar. et Kir.*, purification of the sum of extracted substances (the resin) from worthless products, separation of the received resin on individual components with receiving technical arglabin from which dimethylaminoarglabin hydrochloride is synthesized and subsequent purification, drying and lyophilization thereof, **characterized in that** the extraction of the natural raw material is carried out with using fluid carbon dioxide as an extracting agent at a pressure of 150 $\pm$ 2 · 105 Pa and a temperature of 60°C $\pm$ 0.5, the purification of the resin is carried out by treating it with a water-alcohol mixture in the ratio 1 : 2 at a temperature of 70°C, settling the filtrate in a cooler for 24 hours at temperature of 10 - 15°C with a separation of the filtrate from the residual, subjecting the residual to water-alcohol processing for three times at a ratio of 1 : 2, combining and evaporating the filtrate up to 1/5 starting volume, to the obtained evaporated filtrate ethyl acetate is added in the ratio 1 : 1, the received solution is transferred into a separator and stirred for 5 minutes before complete separation of layers, the resin separation into individual components is carried out by the method of preparative fluid chromatography with application of reversed phase $C_{18}$, for the synthesis of dimethylaminoarglabin hydrochloride, the technical arglabin is solved in acetonitrile at the weight ratio 1 : 10 with the addition of dimethylammonium dimethylcarbamat in the molar ratio 1 : 1.62.

## Patentansprüche

**1.** Verfahren zur Herstellung von 1(10)$\beta$-Epoxy-13-dimethylamino-5,7a,6,11$\beta$(H)-guaia-3(4)-en-6,12-olid-Hydrochlorid, umfassend die Extraktion der endemischen Pflanze *Artemisia glabella Kar. Et Kir.* als natürlichen Ausgangsstoff, Reinigung der Gesamtheit der extrahierten Substanzen (des Harzes) von wertlosen Produkten, Auftrennung des erhaltenen Harzes in die Einzelkomponenten unter Erhalt von technischem Arglabin, aus dem Dimethylaminoarglabin-Hydrochlorid synthetisiert wird, und nachfolgende Reinigung, Trocknung und Lyophilisierung, **dadurch gekennzeichnet, dass** die Extraktion des natürlichen Ausgangsstoffes unter Verwendung von flüssigem Kohlendioxid als Extraktionsmittel bei einem Druck von 150 $\pm$ 2 · $10^5$ Pa und einer Temperatur von 60 °C $\pm$ 0,5 durchgeführt wird, die Reinigung des Harzes durch Behandlung mit einem Wasser-Alkohol-Gemisch bei einem Verhältnis von 1 : 2 bei einer Temperatur von 70 °C durchgeführt wird, wonach man das Filtrat sich in einem Kühler während 24 Stunden bei einer Temperatur von 10 - 15 °C unter Abscheidung des Filtrats vom Rückstand absetzen lässt, diesen mit dem Wasser-Alkohol-Gemisch bei einem Verhältnis von 1 : 2 dreimal behandelt, die Filtrate miteinander vereinigt und bis zu 1/5 des Ausgangsvolumens eindampft, dem erhaltenen eingedampften Filtrat Ethylacetat bei einem Verhältnis von 1 : 1 zusetzt, die erhaltene Lösung in einen Scheider gibt und während 5 Minuten vor der vollständigen Auftrennung der Schichten rührt, die Auftrennung des Harzes in die einzelnen Komponenten nach dem Verfahren der präparativen Flüssigkeitschromatographie mit umgekehrter $C_{18}$-Phase zur Synthese des Dimethylaminoarglabin-Hydrochlorids durchführt und das technische Arglabin in Acetonitril bei einem Gewichtsverhältnis von 1 : 10 unter Zugabe von Dimethylammoniumdimethylcarbamat bei einem Molarverhältnis von 1 : 1,62 löst.

## Revendications

**1.** Procédé de production de chlorhydrate de 1(10)$\beta$-époxy-13-diméthylamino-5,7a,6,11$\beta$(H)-guaia-3(4)-èn-6,12-olide, comprenant l'extraction de la matière brute naturelle de la plante endémique *Artemisia glabella Kar. et Kir.*, la purification de la somme des substances extraites (la résine) des produits sans valeur, la séparation de la résine recueillie sur les composants individuels avec l'arglabine technique receveuse à partir de laquelle le chlorhydrate de diméthylaminoarglabine est synthétisé et sa purification, séchage et lyophilisation subséquents, **caractérisé en ce que** l'extraction de la matière brute naturelle est réalisée en utilisant du dioxyde de carbone liquide comme agent d'extraction à une pression de 150 $\pm$ 2 • $10^5$ Pa et une température de 60 °C $\pm$ 0,5, la purification de la résine est réalisée en la traitant avec un mélange eau-alcool dans le rapport 1 : 2 à une température de 70 °C, en sédimentant le filtrat dans un appareil de refroidissement durant 24 heures à une température de 10 à 15 °C avec une séparation du filtrat de la substance résiduelle, en soumettant la substance résiduelle à un traitement eau-alcool durant trois fois à un rapport de 1 : 2, en combinant et en évaporant le filtrat jusqu'à 1/5 du volume de départ, au filtrat évaporé obtenu de l'acétate d'éthyle est ajouté dans le rapport 1 : 1, la solution recueillie est transférée dans un séparateur et agitée durant 5 minutes avant la séparation complète des couches, la séparation de la résine en composants

individuels est réalisée par le procédé de chromatographie liquide préparative avec l'application de $C_{18}$ en phase inverse, pour la synthèse de chlorhydrate de diméthylaminoarglabine, l'arglabine technique est dissoute dans de l'acétonitrile au rapport en poids de 1 : 10 avec l'addition de diméthylcarbamate de diméthylammonium dans le rapport molaire 1 : 1,62.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KZ 10913
- KZ 5277
- WO 2006012824 A